# EUROPÄISCHE PATENTSCHRIFT

(11) **EP 1 370 306 B1**
(45) Veröffentlichungstag und Bekanntmachung des Hinweises auf die Patenterteilung: **19.10.2005**
(21) Anmeldenummer: 01900128.8
(22) Anmeldetag: 05.01.2001
(51) Int. Cl.: A61L 31/02, A61L 31/14

(54) **DURCH KORROSION ABBAUBARE METALLISCHE MEDIZINISCHE IMPLANTATE**
MEDICAL METAL IMPLANTS THAT CAN BE DECOMPOSED BY CORROSION
IMPLANTS MEDICAUX METALLIQUES DEGRADABLES PAR CORROSION

(43) Veröffentlichungstag der Anmeldung: 17.12.2003
(73) Patentinhaber: Hausdorf, Jacqueline Yvonne, 30938 Burgwedel (DE); Meyer, Jörg, 22297 Hamburg (DE)
(72) Erfinder: HAUSDORF, Gerd, deceased (DE); MEYER, Jörg, 22297 Hamburg (DE); NIEMEYER, Matthias, 30163 Hannover (DE); HEUBLEIN, Eva, 30627 Hannover (DE); TAI, Phan-tan, 30167 Hannover (DE); PEUSTER, Matthias, 32547 Bad Oeynhausen (DE); Hausdorf, Jacqueline Yvonne, 30938 Burgwedel (DE)
(74) Vertreter: Lenzing, Andreas
(86) Internationale Anmeldenummer: PCT/EP2001/000085
(87) Internationale Veröffentlichungsnummer: WO 2002/053202

(56) Entgegenhaltungen:
- EP-A- 0 966 979
- WO-A-95/30384
- DE-A- 19 731 021
- WEILL A. ET AL.: ""Corrosion" of Tungsten Spirals. A Disturbing Finding." INTERVENTIONAL NEURORADIOLOGY, Bd. 4, 1. Januar 1998 (1998-01-01), Seiten 337-340, XP001025780

## Beschreibung

Die vorliegende Erfindung betrifft ein in vivo abbaubares medizinisches Implantat aus einem metallischen Werkstoff aus der Gruppe der Implantate gemäß dem Oberbegriff des Anspruchs 1.

Ein derartiges Implantat ist aus der DE 19731021 bekannt. Bei diesen Implantaten wird praktisch mit der Implantation ein Korrosionsvorgang in Gang gesetzt, der nach einiger Zeit dazu führt, daß das Implantat zunächst mechanisch instabil und dann komplett abgebaut wird. Bei diesen Implantaten soll das Material so gewählt werden, daß die Korrosion zur Aufrechterhaltung der mechanischen Stabilität im erforderlichen Umfang langsam abläuft. Dies bedingt auch einen entsprechend langsamen Abbau des Implantatmaterials, nachdem dieses Implantat seine Aufgabe erfüllt hat. Der vollständige Abbau wird in der Praxis ein mehrfachen der Zeit benötigen, für die die mechanische Funktion aufrecht erhalten bleiben soll.

Das Dokument EP-A 0 966 979 offenbart eine implantierbare, bioresorbierbare Gefäßstütze aus einer Metalllegierung auf Basis der Metalle Magnesium, Titan, Zirkonium, Niob, Tantal, Zink oder Silizium mit einem Zusatz eines zweiten Metalls zur Gewährleistung ausreichender Korrosion der Legierung.

Weiter ist bekannt, sogenannte Coils als Gefäßverschlußsysteme aus einer Wolframlegierung zu fertigen, die korrodieren können. Gerade bei Gefäßverschlußsystemen ist der Abbau des Implantats nicht erwünscht, insbesondere dann nicht, wenn die Implantation nicht zu dem angestrebten Gefäßverschluß geführt hat.

Es ist deshalb Aufgabe der vorliegenden Erfindung, ein Implantat aus der im Oberbegriff des Anspruchs 1 genannten Gruppe dahingehend zu verbessern, daß die mechanische Stabilität des Implantats so lange wie nötig aufrechterhalten bleibt und die Korrosion nach erfüllter mechanischer Funktion einen beschleunigten Abbau des Implantats ermöglicht. Weiter ist es Aufgabe der vorliegenden Erfindung, ein Wirkstoffdepot zu schaffen, das eine gezielt steuerbare Wirkstofffreisetzung ermöglicht.

Diese Aufgabe wird von einer Erfindung mit den Merkmalen des Anspruchs 1 gelöst.

Weil bei dem erfindungsgemäßen Implantat vorgesehen ist, daß der Werkstoff als Hauptlegierungsbestandteil 95% bis 99,5% Wolfram oder Molybdän enthält, wird das Implantat in der biologischen Umgebung, für deren Einsatz es vorgesehen ist, ein pH-Wert-abhängiges Korrosionsverhalten zeigen, wobei der Übergang von einem nicht korrodierenden Zustand in einen korrodierenden Zustand bei einem für das jeweilige biologische System tolerierbaren pH-Wert liegt. Insbesondere wird der Übergang zum korrodierenden Zustand durch einen den pH-Wert im biologischen System steuernden Vorgang beeinflußt.

Im Falle des Wirkstoffdepots wird die Freisetzung des Wirkstoffs durch die Änderung des pH-Werts vom korrosionsinhibierenden Zustand in den korrosionsfördernden Zustand verstärkt.

Nebenbestandteile können dabei eine Vielzahl von Elementen sein, die auch ohne Einfluß auf das Korrosionsverhalten sein können. Bei dem Implantat ist jedoch von Vorteil, wenn der Werkstoff als Nebenbestandteil ein oder mehrere Elemente aus der Gruppe der Lanthanoide, insbesondere Cer, der Actinoide, Eisen, Osmium, Tantal, Rhenium, Gadolinium, Yttrium oder Scandium enthält. Mit diesen Legierungszusätzen kann ein gutes Korrosionsverhalten für den angestrebten Verwendungszweck erreicht werden. Dabei sind typische Zusammensetzungen so gestaltet, daß der Hauptlegierungsbestandteil in dem Werkstoff zu 95 % bis 99,5 % enthalten ist und der Rest an 100 % von dem wenigstens einen Nebenbestandteil gebildet ist. Eine besonders schnelle Zersetzung in einem bestimmten pH-Bereich ist möglich, wenn der Werkstoff eine kristalline Struktur mit Korngrößen von 0,5 µm bis 30 µm, insbesondere 0,5 µm bis 5 µm aufweist. Dann findet eine flächenhafte Korrosion statt. Jedoch kann bei Korngrößen von 10 µm oder mehr auch eine interkristalline Korrosion stattfinden, die zu einer Bildung von Partikeln führt, wobei diese Partikel vom Körper ausgeschieden werden können.

Vorteilhaft ist außerdem, wenn das Implantat außer dem Legierungswerkstoff metallische oder nichtmetallische Einschlüsse nach Art von Sintermetall enthält, die aus einem im wesentlichen reinen Alkali- oder Erdalkalimetall bestehen. Diese Einschlüsse können sowohl die gezielte Korrosion hinsichtlich des Beginns als auch hinsichtlich der Korrosionsgeschwindigkeit fördern. Außerdem können die dann bei der Korrosion freigesetzten Alkali- oder Erdalkaliionen physiologisch vorteilhaft wirksam werden. Eine hinsichtlich der mechanischen Stabilität bei gutem Korrosionsverhalten vorteilhafte Ausführungsform ergibt sich, wenn das Implantat einen im wesentlichen rohrförmigen Grundkörper aufweist.

Im folgenden wird ein Ausführungsbeispiel der vorliegenden Erfindung beschrieben.

Ein kardiovaskulärer Stent wird aus einem rohrförmigen Grundkörper aus Wolfram oder einer Wolframlegierung in an sich bekannter Weise gefertigt. Der Stent wird in ein verengtes Blutgefäß eingeführt und im Bereich der Gefäßverengung dilatiert. Dort verbleibt der Stent, bis das Gefäß erneut ausreichende Eigenstabilität gewonnen hat. Bis zu diesem Zustand wird der pH-Wert im Blut des Patienten durch regelmäßige Gabe von Ascorbinsäure auf einem Wert von < pH 7,4 gehalten. Sobald entschieden ist, daß die Stützfunktion des Stents nicht länger benötigt wird, wird die Gabe von Ascorbinsäure abgesetzt und das Blut des Patienten auf einen pH-Wert oberhalb von 7,4 alkalisiert, was durch Gabe von Diuretika erfolgt. In dem geänderten Milieu wird der Stent relativ schnell korrodieren. Es ergibt sich eine relativ schnelle Auflösung des Materials, wobei die Anordnung des Materials in dem Blutgefäß für eine schnelle Abfuhr der entstehenden Wolframpartikel oder Wolframionen führt und so verhindert, daß eine lokal toxische Konzentration entstehen kann.

Als Werkstoff wird in diesem Ausführungsbeispiel eine Legierung von 99,2% W und 0,8% Ce mit einer Korngröße von etwa 1 µm verwendet. Im menschlichen Blutstrom ergibt sich dabei eine flächenhafte Korrosion, deren Abbaugeschwindigkeit bei einem pH.Wert von 7,2 20 µm pro Jahr beträgt. Durch Anhebung des pH-Wertes auf 7,4 wird die Abbaugeschwindigkeit auf 50 µm/a gesteigert.

Bei einem zweiten Ausführungsbeispiel wird ein Wirkstoffdepot aus einer Wolframlegierung gefertigt, wobei in das Legierungsmaterial nach Art von Sintermetall Wirkstoffe mit therapeutisch wirksamen Eigenschaften eingebracht sind (Metallionen, Medikamente, mRNA, Vektoren). Das Implantat wird an einer zu behandelnden Position des Biosystems außerhalb des Blutstroms angeordnet.

Wie im vorherigen Ausführungsbeispiel wird das Biosystem durch Gabe von Ascorbinsäure oder ähnlichen Wirkstoffen zunächst auf einem relativ niedrigen pH-Wert gehalten. Sobald die Wirkstoffe benötigt werden, wird eine alkalisierende Substanz zugeführt, so daß der pH-Wert ansteigt. Die einsetzende Korrosion setzt das therapeutisch wirksame Material frei und führt, da es außerhalb des Blutstroms angeordnet ist, zu einer hohen lokalen Wirkstoffkonzentration, die therapeutisch wirksam ist, ohne das übrige Biosystem zu beeinträchtigen. Auf diese Weise können Tumore, Gefäßeinengungen durch Intimaproliferation, andere Gefäßreaktionen wie Fibrosen, aber auch Infektionen oder dergleichen durch gezielt wählbare lokale und systemische Wirkstoffdosierungen bekämpft werden.

Ähnliches gilt für Implantate in den Harnwegen oder den Gallenwegen, wobei für die Steuerung der Wirkstofffreisetzung bei diesen Anwendungsfällen ein breiteres pH-Spektrum zur Verfügung steht. Hier ist nach einem weiteren Ausführungsbeispiel vorgesehen, daß ein Harnwegsstent aus einer Legierung von 98,5% Mo mit 1,5% Tantal gefertigt ist. Dieser Stent ist bei einem pH-Wert von mehr als 2 stabil, während durch eine Anderung des pH-Wertes auf unter 2 der korrosive Abbau beschleunigt wird. Außer Tantal kommen hier Platin und Gold als Nebenbestandteile in Betracht.

Wie im ersten Ausführungsbeispiel können auch chirurgische Clips, metallisches Nahtmaterial oder dergleichen so lange stabil an ihrem Ort gehalten haben, bis sie ihre Funktion erfüllt haben. Danach kann durch gezielte Änderung des pH-Werts die Korrosion und damit die Auflösung des Materials induziert werden.

## Patentansprüche

1. In vivo abbaubares medizinisches Implantat aus der Gruppe, die folgendes umfaßt:
Stents (Koronarstents, Peripheriestents, Trachealstents, Gallengangsstents, Ösophagusstents), chirurgische Clips, Osteosynthesematerial, biologische Matrix (Schaum), Metallgeflecht, Metallfäden, Wirkstoffdepots,
aus einem metallischen Werkstoff, **dadurch gekennzeichnet, daß** der Werkstoff als Hauptlegierungsbestandteil 95% bis 99,5% Wolfram oder 95% bis 99,5% Molybdän enthält.

2. Medizinisches Implantat nach Anspruch 1, **dadurch gekennzeichnet, daß** der Werkstoff als Nebenbestandteil ein oder mehrere Elemente aus der Gruppe enthält, die folgendes umfaßt: Lanthanoide, insbesondere Cer, Actinoide, Eisen, Osmium, Tantal, Platin, Gold, Rhenium, Gadolinium, Yttrium, Scandium.

3. Medizinisches Implantat nach einem der vorhergehenden Ansprüche, **dadurch gekennzeichnet, daß** der Werkstoff eine kristalline Struktur mit Korngrößen von 0,5 µm bis 30 µm, insbesondere 0,5 µm bis 5 µm aufweist.

4. Medizinisches Implantat nach einem der vorhergehenden Ansprüche, **dadurch gekennzeichnet, daß** das Implantat außer dem Werkstoff metallische oder nichtmetallische Einschlüsse enthält, die aus einem im wesentlichen reinen Alkali- oder Erdalkalimetall, einem Medikament, mRNA oder einem Vektor bestehen.

5. Medizinisches Implantat nach einem der vorhergehenden Ansprüche, **dadurch gekennzeichnet, daß** das Implantat einen im wesentlichen rohrförmigen Grundkörper aufweist.

## Claims

1. An in vivo degradable medical implant from the group comprising the following:
stents (coronary stents, peripheral stents, tracheal stents, bile-duct stents, oesophageal stents), surgical clips, osteosynthesis material, biological matrix (foam), metal braid, metal filaments, active-substance depots,
made of a metallic material, **characterised in that** the material contains 95 % to 99.5 % tungsten or 95% to 99.5% molybdenum by way of principal alloy constituent.

2. Medical implant according to Claim 1, **characterised in that** the material contains by way of secondary constituent one or more elements from the group comprising the following: lanthanoids, in particular cerium, actinoids, iron, osmium, tantalum, platinum, gold, rhenium, gadolinium, yttrium, scandium.

3. Medical implant according to one of the preceding claims, **characterised in that** the material exhibits a crystalline structure with grain sizes from 0.5 µm to 30 µm, in particular 0.5 µm to 5 µm.

4. Medical implant according to one of the preceding claims, **characterised in that** the implant contains, in addition to the material, metallic or non-metallic inclusions that consist of a substantially pure alkali metal or alkaline-earth metal, a medicament, mRNA or a vector.

5. Medical implant according to one of the preceding claims, **characterised in that** the implant has a substantially tubular structure.

## Revendications

1. Implant médical dégradable in vivo, appartenant au groupe qui comprend :
les stents (stents coronaires, stents périphériques, stents trachéaux, stents biliaires, stents dans l'oesophage), les agrafes chirurgicales, le matériel d'ostéosynthèse, la matrice biologique (mousse), treillis métallique, fils métalliques, dépôts de principe actif,
constitué en matériau métallique, **caractérisé en ce que** le matériau, comme composant principal en alliage, contient de 95 % à 99,5 % de tungstène ou 95 % à 99,5 % de molybdène.

2. Implant médical selon la revendication 1,
**caractérisé en ce que** le matériau, comme composant secondaire, contient un ou plusieurs éléments du groupe qui comprend : les lanthanides, en particulier le cérium, l'actinide, le fer, l'osmium, le tantale, le platine, l'or, le rhénium, le gadolinium, l'yttrium, le scandium.

3. Implant médical selon l'une des revendications précédentes, **caractérisé en ce que** le matériau présente une structure cristalline avec une grosseur de cristaux comprise entre 0,5 µm et 30 µm, en particulier entre 0.5 µm et 5 µm.

4. Implant médical selon l'une des revendications précédentes, **caractérisé en ce que** l'implant, hormis le matériau dont il est constitué, contient des inclusions métalliques ou non-métalliques, constituées par un métal alcalin ou alcalinoterreux sensiblement pur, par un médicament, par un ARN messager, ou par un vecteur.

5. Implant médical selon l'une des revendications précédentes, **caractérisé en ce que** l'implant présente un corps de base sensiblement tubulaire.
